# EUROPÄISCHE PATENTANMELDUNG

(11) **EP 1 386 558 A1**
(43) Veröffentlichungstag der Anmeldung: **04.02.2004**
(21) Anmeldenummer: 03003972.1
(22) Anmeldetag: 22.02.2003
(51) Int. Cl.: A45D 7/00

(54) **Verfahren zur Erstellung, Pflege und Wiederentfernung von temporären Rasta-Frisuren**

(30) Priorität: 31.07.2002 DE 10234804
(71) Anmelder: Wella Aktiengesellschaft, 64274 Darmstadt (DE)
(72) Erfinder: Maillefer, Sarah, Dr., 1749 Middes (CH); Kalbfleisch, Axel, 64295 Darmstadt (DE); Rehmann, Andre, 3185 Schmitten (CH); Chambettaz, Daniel, 1717 St. Ursen (CH)

(57) **Zusammenfassung**

Es wird ein Verfahren zur Erstellung, Pflege und Wiederentfernung von temporären Rasta-Frisuren beschrieben. Das Verfahren enthält einen oder mehrere der folgenden Schritte: Aufbringen eines die Rauheit der Haaroberfläche erhöhenden Stoffes auf die Haare, Aufbringen eines die Haftung der Haare aneinander erhöhenden Stoffes auf die Haare, Erstellen temporärer Rastasträhnen durch strähnchenweises Verkletten der Haare. Die Reinigung der temporären Rasta-Frisur erfolgt vorzugsweise unter Verwendung eines konkaven Schwammes, ohne die Frisur zu zerstören. Zur Wiederentfernung der temporären Rasta-Frisur wird eine die Naßkämmbarkeit erhöhende Zusammensetzung auf das Haar aufgetragen und die Rastasträhnen werden geöffnet und entwirrt. Gegenstand der Erfindung sind auch Mehrkomponenten-Kits zur Durchführung des Verfahrens.

## Beschreibung

Gegenstand der Erfindung ist ein Verfahren zur Erstellung, Pflege und Entfernung von temporären Rasta-Frisuren sowie hierfür benötigte Zusammensetzungen, Komponenten und Kits.

Eine permanente Rasta-Frisur ist eine Haartracht aus eng geflochtenen langen Zöpfen aus in der Regel dünnen und extrem stark verfilzten Haarsträhnen. Derartige Frisuren werden auch als Dreadlocks oder Dreads bezeichnet, die Begriffe werden im Folgenden synonym verwendet. Ethnische Haare (z.B. afrikanisches oder afro-amerikanisches Haar, sogenanntes kinky hair) bietet aufgrund der natürlichen Beschaffenheit und der natürlichen Krause ideale Voraussetzungen zum Erstellen von Dreadlocks bzw. Rasta-Frisuren. Mit geeigneten Techniken sind Rasta-Frisuren aber auch bei anderen, u.a. auch glatten Haarqualitäten, z.B. mitteleuropäischem oder asiatischem Haar zu verwirklichen. Hierfür ist es aber erforderlich, vor der Lockenerstellung eine zur starken Verfilzung neigende Haarstruktur zu erzeugen. Dies erfolgt üblicherweise durch eine Kombination von chemischer und mechanischer Behandlung des Haares. Zunächst ist es erforderlich, dass das Haar unnatürlich stumpf gemacht wird und dass die Haarschuppen nicht am Haarschaft anliegen sondern möglichst weit abstehen. Die chemische Behandlung kann mit einem Haarreduktionsmittel erfolgen, wie es bei Dauerwellbehandlungen eingesetzt wird (z.B. Thioverbindungen wie Thioglykolsäure), mit dem Unterschied, dass Wirkstoffkonzentration und/oder Einwirkzeit extrem gesteigert werden. Hierbei wird die Cuticulaschicht des Haares vermutlich ganz oder teilweise entfernt um die notwendige Neigung zum Verfilzen zu erreichen. Eine andere chemische Behandlung ist diejenige mit einer stark alkalischen Zusammensetzung, z.B. Kernseife oder einem alkalischen Shampoo ohne jeglichen Pflegezusatz. Weiterhin ist eine intensive mechanische Behandlung des Haares zur Abspreizung der Schuppen erforderlich. Dies kann durch intensives, bis zu mehrere Stunden dauerndes, so fest wie möglich erfolgendes Toupieren der Haare mit einem sehr feinen Kamm, z.B. mit einem Läusekamm oder ähnlichem, in der Regel mit stabilen Metallzinken, erfolgen. Toupieren wird auch als back combing bezeichnet. Diese Begriffe werden im Folgenden synonym verwendet. Hierbei kommt es zu einer intensiven, irreversiblen Verfilzung der Haare und das Haar wird irreparabel geschädigt. Ein weiteres Problem neben der Haarschädigung ist, dass die mit den herkömmlichen Techniken erstellten permanenten Rasta-Frisuren nicht wieder entfernbar sind und die Verfilzung der Haare irreversibel ist, d.h. nicht ohne weiteres wieder rückgängig gemacht werden kann. Wer die Frisur einmal hat, wird sie in der Regel nur noch mit einer Schere durch Abschneiden der Haare wieder los. Für modebewußte Anwender, die eine Rasta-Frisur nur vorübergehend oder nur zu einem bestimmten Anlaß, z.B. für eine Party am Wochenende tragen möchten, am nächsten Tag aber ihre ursprüngliche Frisur ohne Schädigung der Haarstruktur zurück haben möchten, besteht daher ein starkes Bedürfnis nach einer Technik zur Erstellung einer wieder entfernbaren, temporären Rasta-Frisur ohne haarschädigende Behandlungsschritte. Ein weiteres Problem mit herkömmlich erstellten Rasta-Frisuren ist ein hygienisches und besteht darin, dass eine Reinigung innerhalb der stark verfilzten Strähnen nur schwer oder gar nicht möglich ist. Es besteht deshalb die Gefahr, dass sich die verfilzten Strähnen von innen heraus zersetzen können, es zu geruchlichen Beeinträchtigungen kommen kann und einzelne Strähnen sogar abbrechen oder ausfallen können. Für Anwender, die über einen längeren Zeitraum eine Rasta-Frisur tragen wollen, ist deshalb eine Technik wünschenswert, die es erlaubt, die Strähnen einer Rasta-Frisur nach einer gewissen Zeit wieder öffnen zu können und nach intensiver Reinigung und/oder Pflege der Haare die gewünschte Rasta-Frisur erneut reversibel erstellen zu können.

Es wurde nun gefunden, dass die Aufgabe gelöst wird durch das nachstehend näher beschriebene Verfahren zur Erstellung, Pflege und Wiederentfernung von temporären Rasta-Frisuren. Das erfindungsgemäße Verfahren umfaßt einen oder mehrere der folgenden Schritte: Aufbringen eines die Rauheit der Haaroberfläche erhöhenden Stoffes oder mindestens einer das Toupieren der Haare erleichternden Zusammensetzung auf die Haare, Aufbringen eines die Haftung der Haare aneinander erhöhenden Stoffes auf die Haare und Erstellen temporärer Rastasträhnen durch strähnchenweises Verkletten der Haare, ohne die Haare wesentlich, d.h. irreversibel zu verfilzen. Das Verkletten kann z.B. erfolgen entweder durch leichtes Toupieren (back combing), oder durch ein- oder mehrfaches Verdrehen, Teilen und Auseinanderziehen der geteilten Strähnchen, oder durch eine Kombination von Verdrehen der Strähnchen über die Längsachse und leichtem Toupieren der verdrehten Strähnchen. Die Reinigung der temporären Rasta-Frisur kann unter Verwendung eines konkaven Schwammes erfolgen, ohne die Frisur zu zerstören. Zur Wiederentfernung der temporären Rasta-Frisur wird eine die Naßkämmbarkeit erhöhende Zusammensetzung auf das Haar aufgetragen und die Rastasträhnen werden geöffnet und/oder entwirrt, vorzugsweise mechanisch unter Verwendung eines gewöhnlichen Kamms oder einer Bürste.

Überraschend war, dass es möglich ist, einen authentisch oder zumindest nahezu authentisch aussehenden Rasta-Look, d.h. ein Aussehen wie "echte", permanente Rastas zu erzeugen, ohne die Haare zu schädigen und ohne die bisher als Voraussetzung angesehene starke, irreversible Verfilzung der Haare. überraschend war außerdem, dass die Wiederentfernung der Rasta-Frisur auf einfachem Wege problemlos möglich ist und die Haare nach der Wiederentfernung der Frisur teilweise sogar einen noch gepflegteren und gesünderen Eindruck, insbesondere hinsichtlich Griff, Glanz und Kämmbarkeit machen, als vor der Behandlung. Dies ist gerade deshalb erstaunlich, da bisher eine Rasta-Frisur fast schon als Synonym für stark geschädigte und ungepflegte Haare ist und nun gewissermaßen das Gegenteil erreicht werden kann. Die Pflegewirkung geht dabei teilweise noch darüber hinaus, was man von der Wirkung der unten näher beschriebenen Remover-Zusammensetzung C erwartet hätte. Dies deutet auf eine synergistische Pflegewirkung der Remover-Zusammensetzung C in Kombination mit den nachfolgend beschriebenen Vorbehandlungs-Zusammensetzungen A und/oder B hin.

Gegenstand der Erfindung ist ein Verfahren zur Erstellung einer temporären Rasta-Frisur, wobei vor, während oder nach der Frisurenerstellung mindestens ein Verfahrensschritt erfolgt, der ausgewählt ist aus (1) Aufbringen mindestens eines die Rauheit der Haaroberfläche erhöhenden Stoffs auf die Haaroberfläche oder mindestens einer das Toupieren der Haare erleichternden Zusammensetzung auf das Haar und (2) Aufbringen mindestens eines die Haftung der Haare aneinander erhöhenden Stoffes auf das Haar und/oder auf die Haarspitzen. Unter einer temporären Rasta-Frisur wird eine Frisur verstanden mit dem optischen Eindruck eines Rasta-Looks, wobei die Frisur im Gegensatz zu einer permanenten Rasta-Frisur wieder entfernbar ist und wobei insbesondere die Haare im Gegensatz zu einer permanenten Rasta-Frisur nicht oder zumindest nicht wesentlich verfilzt sondern lediglich verklettet sind, d.h. miteinander verschlungene Haare wieder mechanisch separiert werden können. Erfindungsgemäß ist auch eine Frisur, bei der nicht sämtliche Haare sondern nur einzelne Strähnchen erfindungsgemäß behandelt und verklettet sind (partielle Rasta-Frisur). Ein die Rauheit der Haaroberfläche erhöhender Stoff ist ein Stoff, welcher auf der Haaroberfläche haftet und bewirkt, dass sich das Haar gegenüber dem nicht behandelten, sauberen Haar stumpf oder rau anfühlt. Dieser Stoff kann mittels einer unten näher beschriebenen Zusammensetzung A auf das Haar aufgebracht werden.

Die Stabilität der temporären Rasta-Frisur wird deutlich erhöht, wenn auf die mit dem die Rauheit erhöhenden Stoff vorbehandelten Haare zusätzlich ein die Haftung der Haare aneinander erhöhender Stoff auf das Haar und/oder auf die Haarspitzen aufgebracht wird. Die Aufbringung des die Haftung der Haare erhöhenden Stoffes kann mittels einer unten näher beschriebenen Zusammensetzung B erfolgen und kann gegebenenfalls vor und/oder nach der Erstellung der Rastasträhnen stattfinden.

### Erstellung der temporären Rasta-Frisur

Zur Erstellung der temporären Rasta-Frisur werden aus den erfindungsgemäß vorbehandelten Haaren ein oder mehrere temporäre Rastazöpfe (Dreadlocks) erstellt. Dies kann durch mehrere Methoden erfolgen. Empfohlen wird mindestens eine der drei nachfolgend beschriebenen Techniken, wobei die Empfehlungen als beispielhafte Ausführungsformen zu verstehen sind. Bei allen Techniken wird mit der Dreadlock Erstellung im Nacken begonnen. Dazu wird eine Sektion quer zum Kopf (z.B. von ca. 3 cm Stärke), von Seite zu Seite abgeteilt. Die Grundfläche der einzelnen Dreadlocks (Einzelsträhnen) ist von der gewünschten Stärke/Dicke abhängig, beträgt in aller Regel 6 - 9 cm² und kann quadratisch, rechteckig trapezförmig oder dreieckig ausgeführt werden. In dieser Technik arbeitet man sich in Richtung Vorderkopf, wobei die Dreadlocks dachziegelartig übereinander zum Liegen kommen. Um den geplanten Frisurenfall zu begünstigen, werden die Dreadlocks bei ihrer Erstellung vorzugsweise direkt in die gewünschte Richtung gehalten.

### Besonders bevorzugte Methode:

Die abgeteilte Einzelsträhne wird mit einem geeigneten Toupierkamm gleichmäßig vom Haaransatz zur Haarspitze hin leicht toupiert (verklettet, nicht verfilzt). Während des Toupierens wird die Einzelsträhne über ihre Längsachse gedreht, wodurch ein besonders runder und "echt" wirkender Dreadlock entsteht. Anstelle eines Toupierkamms können auch gleichwirkende Hilfsmittel wie z.B. Bürsten oder Striegel mit Borsten, Zähnen oder Zinken aus Metall, Hartkunststoff oder ähnlichem verwendet werden.

### Weitere, bevorzugte Methode:

Die abgeteilte Einzelsträhne wird mit den Fingern kräftig über ihre Längsachse gedreht. Nach 2-3 Umdrehungen wird die Haarsträhne an der Spitze willkürlich geteilt. Die beiden durch die Teilung entstandenen Haarsträhnen werden nun auseinandergezogen, wodurch sich die zuvor erstellten 2-3 Umdrehungen zum Haaransatz hin bewegen und sich dabei verkletten (ähnlich dem Toupiervorgang). Das Drehen, Teilen und Auseinanderziehen der Haarsträhne wird solange durchgeführt, bis sich die gesamte Länge der Einzelsträhne verklettet hat.

### Weitere, mögliche Methode

Die abgeteilte Einzelsträhne wird mit den Fingern so lange kräftig über ihre Längsachse gedreht, bis die gesamte Strähne "gekordelt" wirkt. Die Haarspitzen der Einzelsträhne werden festgehalten, und der Zug auf die Längsachse etwas verringert. Nun wird mit einem geeigneten Toupierkamm in die bereits gedrehte Strähne leicht hineintoupiert, ohne das Haar zu verfilzen. Das Toupieren erfolgt über die gesamte Längsachse der Einzelsträhne. Danach wird die gedrehte und toupierte Strähne zwischen Daumen und Zeigefinger nochmals gerollt um den verkletteten Effekt zu verstärken.

### Zusammensetzung A

Die Aufbringung des die Rauheit der Haaroberfläche erhöhenden Stoffes kann mittels einer ersten Zusammensetzung A erfolgen. Geeignete Stoffe sind z.B. Stoffe, die bei Raumtemperatur (25°C) fest sind und in Form von Partikeln vorliegen können. Geeignet sind etwa Silica, Silikate, Aluminate, Tonerden, Mica, Salze, insbesondere anorganische Metallsalze, Metalloxide, z.B. Titandioxid, Minerale und Polymer. Geeignet sind auch Polymere, die dem Haar einen rauhen, stumpfen Griff verleihen, festellbar durch Halbseitenversuche von polymerbehandeltem Haar gegenüber unbehandeltem Haar.

Geeignete raue Polymere sind beispielsweise Copolymere aus Alkylacrylamiden, insbesondere mit C1- bis C8-Alkylgruppen und mindestens einem Monomer, ausgewählt aus Acrylsäure, Methacrylsäure und deren einfachen Estern, insbesondere den C1- bis C4-Alkylestern (INCI: Acrylates/Octylacrylamide Copolymer, Acrylates/t-Butylacrylamide Copolymer z.B. Amphomer® HC bzw. Ultrahold® 8); Copolymere aus Vinylmethylether und Alkylhalbestern von Maleinsäure, insbesondere den C1- bis C5-Alkylhalbestern (INCI: Butylester of PVM/MA Copolymer, z.B. Gantrez® ES 425; Copolymere aus Vinylacetat und Crotonsäure (INCI: VA/Crotonates Copolymer, z.B. Luviset® CA 66).

Der die Rauheit bewirkende Stoff kann in der Zusammensetzung A in fester, partikelförmiger Form vorliegen und ist dann vorzugsweise stabil dispergiert oder er kann in gelöster Form in einem geeigneten, kosmetisch akzeptablen Lösungsmittel vorliegen und scheidet sich dann nach Aufbringen der Lösung auf das Haar und Verdampfen des Lösungsmittels auf dem Haar in fester Form ab. Eine stabile Dispergierung kann erreicht werden, indem die Zusammenstzung A mit einer Fließgrenze versehen wird, die groß genug ist, um ein Absinken der Feststoffpartikel zu verhindern. Eine ausreichende Fließgrenze kann durch Verwendung von geeigneten Gelbildnern in geeigneter Menge eingestellt werden.

Besonders bevorzugte, die Rauheit der Haare bewirkende Stoffe sind Silica (Kieselgel, Siliciumdioxid) und Metallsalze, insbesondere anorganische Metallsalze, wobei Silica besonders bevorzugt ist. Metallsalze sind z.B. Alkali- oder Erdalkalihalogenide wie Natriumchlorid oder Kaliumchlorid; Alkali- oder Erdalkalisulfate wie Natriumsulfat oder Magnesiumsulfat oder Aluminiumhydroxyhalogenide, z.B. Aluminiumhydroxychlorid Al₂(OH)₅Cl. Falls wasserlösliche Metallsalze eingesetzt werden, ist Zusammensetzung A vorzugsweise eine wässrige Lösung dieser Salze und wird bevorzugt in Kombination mit einer Sprühvorrichtung, z.B. einer mechanisch betriebenen Sprühpumpe oder als Aerosolspray eingesetzt. Falls unlösliche Feststoffpartikel eingesetzt werden, die in Wasser, Alkohol und/oder Wasser/Alkohol-Gemischen nicht löslich sind, wie z.B. Siliciumdioxid oder Metalloxide, so ist Zusammensetzung A vorzugsweise ein eine Fließgrenze aufweisendes Gel und enthält zusätzlich mindestens einen die Fließgrenze bewirkenden Gelbildner oder Zusammensetzung A ist eine aufschäumbare Zusammensetzung, enthaltend zusätzlich mindestens ein schaumbildendes Tensid und/oder schaumbildendes Polymer. Die Verschäumung kann durch ein Aerosol-Treibmittel oder mittels einer mechanisch betriebenen Vorrichtung zum Verschäumen erfolgen.

Falls Zusammensetzung A als Gel vorliegt, kommen als Verdicker Polymere in Betracht, die der Zusammensetzung ein plastisches oder pseudoplastisches Verhalten verleihen. Das rheologische Fließverhalten des erfindungsgmäßen Gels ist durch die Existenz einer Fließgrenze charakterisiert, welche vorzugsweise mindestens 3 Pascal beträgt, gemessen mit einem Haake-Rotationsviskosimeter RV 12, Meßsystem PKV-0.5 bei 30°C und bei linear steigendem Schergefälle von 0 bis 100 s⁻¹. Das Gel weist vorzugsweise eine nach Überschreiten der Fließgrenze meßbare Viskosität von 1.000 bis 100.000 mPa s, vorzugsweise von 5.000 bis 50.000 bei 25°C auf, gemessen mit einem Haake Rotationsviskosimeter Typ VT 501 bei einer Schergeschwindigkeit von 12,9 pro Sekunde. Die Fließgrenze ist in Abhängigkeit von Gewicht und Oberfläche der Partikel so gewählt, dass sie mindestens so groß ist wie der durch die Partikel ausgeübte Druck. Hierdurch wird ein Absinken der Partikel verhindert. Die Verdicker sind vorzugsweise in einer Menge von 0,05 bis 10, besonders bevorzugt von 0,1 bis 4 Gew.% enthalten. Die jeweilige optimale Konzentration wird in Abhängigkeit vom Verdickertyp und der Schwere der Partikel gewählt. Geeignete Verdicker sind vernetzte oder nicht vernetzte Polyacrylsäure oder Polymethacrylsäure. Von den Verdickern, die in dem erfindungsgemäßen Mittel enthalten sein können, sind Homopolymere der Acrylsäure mit einem Molekulargewicht von 2.000.000 bis 6.000.000 zu nennen, die z.B. von der Firma BF Goodrich/USA unter der Handelsbezeichnung Carbopol® vertrieben werden. Weitere Verdicker sind z.B. die von der Firma BF Goodrich unter dem Handelsnamen Carbopol® ETD 2001 oder von der Firma Protex/Frankreich unter dem Handelsnamen Modarez® V 600 PX vertriebene Acrylsäurehomopolymer, Acrylsäure/Acrylamid Copolymere oder Sclerotium Gum. Besonders bevorzugt sind die Copolymere der Acrylsäure oder der Methacrylsäure, wie sie z.B. unter dem Handelsnamen Carbopol® 1342 oder Pemulen® TR1 der Firma GOODRICH, USA vertrieben werden. Weitere geeignete Verdicker sind Guar Gum, Xanthan Gum, Bentonite, Hectorite. In einer vorteilhaften Ausführungsform können neben den genannten Verdickern, die der Zusammensetzung eine ausreichende Fließgrenze verleihen, zusätzlich auch solche Verdicker enthalten sein, die der Zusammensetzung in dem für Gele typischen Viskositätsbereich keine ausreichende Fließgrenze verleihen. Derartige Verdicker sind insbesondere Cellulosen und Cellulosederivate wie z.B. Carboxymethylcellulose, Celluloseether und Hydroxyalkylcellulosen, beispielsweise Hydroxyethyloder Hydroxypropylcellulose. Enthalten die Verdicker Säuregruppen, so sind die Säuregruppen vorzugsweise zumindest teilweise mit kosmetisch verträglichen Basen neutralisiert. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin oder Monoethanolamin und Ammoniak, NaOH und andere.

Zusammsetzung A kann auch in Form eines als Aerosol oder Non-Aerosol verschäumbaren oder versprühbaren Produktes vorliegen. Die Aerosol-Produkte liegen in Kombination mit einer druckfesten Aerosolverpackungseinheit mit einem Sprüh- oder einem Schaumkopf vor, enthaltend eine versprüh- oder verschäumbare Zusammensetzung. Die druckfeste Aerosolverpackung des erfindungsgemäßen Aerosol Sprüh-Wachsproduktes kann aus einem beliebigen, für Aerosol Sprüh- oder Schaumprodukte bekanntem Material gefertigt sein. Geeignete Materialien sind insbesondere Metalle wie Aluminium oder Weissblech. Als Sprüh- oder Schaumköpfe können für das erfindungsgemäße Aerosol Sprüh-Wachsprodukt handelsübliche Sprühköpfe und handelsübliche Schaumköpfe verwendet werden. Aerosol-Treibmittel sind vorzugsweise in einer Menge von 1 bis 20, besonders bevorzugt von 2 bis 10 Gew.% enthalten. Als Treibmittel sind beispielsweise niedere Alkane, z.B n-Butan, i-Butan, Propan, Butan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.

Vorzugsweise wird in einem wässrigen oder in einem wässrigalkoholischen Medium mit vorzugsweise mindestens 10 Gew.% Wasser konfektioniert. Das Lösungsmittelsystem ist vorzugsweise in einer Menge von 50 bis 98, besonders bevorzugt von 75 bis 95 Gew.% enthalten. Als Alkohole können insbesondere die für kosmetische Zwecke üblicherweise verwendeten niederen Alkohole mit 1 bis 4 C-Atomen, z.B. Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol.

Im Falle von Non-Aerosol Sprays und Non-Aerosol Schäumen, liegen die Zusammensetzungen in Kombination mit mechanisch betriebenen Vorrichtungen zum Versprühen bzw. Verschäumen vor.

Die verschäumbaren Produkte enthalten in der Regel mindestens eine schaumerzeugende Substanz, z.B. ein schaumbildendes Tensid und/oder ein schaumbildendes Polymer. Die Tenside können nichtionischen, anionischen oder amphoteren Charakter haben. Bevorzugt sind insbesondere nichtionische, schaumerzeugende Tenside. Die Tenside können einzeln oder in einem Gemisch eingesetzt werden. Die Menge an Tensiden kann variieren und ist so gewählt, dass sich eine ausreichende Menge eines gut in das Haar einarbeitbaren Schaumes bildet, wenn die Zusammensetzung aus der Aerosolverpackung bzw. aus dem Produktspender ausgebracht wird. Die Tensidmenge beträgt typischerweise von 0,01 bis 5 Gew.%, besonders bevorzugt von 0,1 bis 2 Gew.%. Geeignete nichtionische Tenside sind beispielsweise C8- bis C18-Fettalkohole, die mit 8 bis 45 mol Ethylenoxid ethoxyliert sein können, z.B. mit bis zu 40 Mol Ethylenoxid pro Mol Fettalkohol oxethylierter Lauryl-, Tetradecyl-, Cetyl-, Oleyl- oder Stearylalkohol, allein oder im Gemisch; hydriertes, mit 8 bis 45 mol Ethylenoxid ethoxyliertes Rizinusöl, C8- bis C18-Fettsäurealkanolamide; die Fettalkohole von oxethyliertem Lanolin oder oxethyliertes Lanolin; Polyglycerylether von gesättigten oder ungesättigten Fettalkoholen und Alkylphenolen mit 8 bis 30 C-Atomen im Alkylrest und 1 bis 10 Glyceryleinheiten im Molekül; Polyethylen/Polypropylen Blockcopolymere und oxethylierte Sorbitanfettsäureester. Besonders bevorzugt als nicht-ionische Tenside sind außerdem Derivate natürlicher Tenside, z.B. Alkylpolyglykoside. Geeignete anionische Tenside sind z.B. Alkylsulfate, Alkylethersulfate, Alkylsulfonate, Alkoylsarcosinate, Alkylisethionate oder Dialkylsulfosuccinate wobei die Alkylgruppen 8 bis 18 C-Atome enthalten können. Geeignete amphotere Tenside sind insbesondere solche vom Betaintyp. Beispiele für Betaine umfassen C₈- bis C₁₈-Alkylbetaine, wie Cocodimethylcarboxymethylbetain, Lauryldimethylcarboxymethylbetain, Lauryldimethyl-alpha-carboxyethylbetain, Cetyldimethylcarboxymethylbetain, Oleyldimethyl-gamma-carboxypropylbetain und Laurylbis-(2-hydroxypropyl)alphacarboxyethylbetain; C₈- bis C₁₈-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C₈- bis C₁₈-Alkyldimethylammoniumacetate, die C₈- bis C₁₈-Alkyldimethylcarboxylmethylammoniumsalze sowie die C₈- bis C₁₈-Fettsäurealkylamidobetaine, z.B. das Kokosfettsäureamidopropylbetain und das N-Kokosfettsäureamidoethyl-N-[2-(carboxymethoxy)ethyl]glycerin (CTFA-Name: Cocoamphocarboxyglycinate).

Die Erleichterung des Toupierens der Haare kann auch durch Behandlung mit Zusammensetzungen erreicht werden wie z.B. Kernseife oder haarkeratinreduzierende Verbindungen enthaltenden Dauerwellmitteln. Die Verwendung von Dauerwellmitteln erfolgt dabei vorzugsweise unter milden Bedingungen, bei denen die Haare nicht stark geschädigt werden, z.B. mit geringer Wirkstoffkonzentration und/oder kurzen Einwirkzeiten. Die Wirkung des Dauerwellmittels beruht darauf, dass die Cystin-Disulfid-Brücken des Haarkeratins durch Einwirken eines Mittels, welches einen haarkeratinreduzierenden Wirkstoff enthält (Verformungsmittel), geöffnet werden. Reduzierende Wirkstoffe sind insbesondere Thioverbindungen, z.B. Salze der schwefeligen Säure oder bestimmte Mercaptoverbindungen, insbesondere Salze oder Ester von Mercaptocarbonsäuren. Als klassisches Dauerwellreduktionsmittel kann die Thioglykolsäure, z.B. als Ammonium- oder Monoethanolaminsalz, verwendet werden. Weitere übliche reduzierende Wirkstoffe sind anorganische Sulfite, 2-Mercaptopropionsäure (Thiomilchsäure), 3-Mercaptopropionsäure, Mercaptocarbonsäureester, Mercaptocarbonsäureamide, Cystein und Derivate dieser Verbindungen. Bevorzugt sind die Salze oder Derivate von Mercaptocarbonsäuren, insbesondere Thioglykolsäure, Cystein und Thiomilchsäure oder deren Salze.

Die Dauerwellwirkstoffe können in einer Menge von 2 bis 30 Gew.% eingesetzt werden, wobei eher niedrige Konzentrationen bis maximal 20 Gew.%, insbesondere maximal 10 Gew.% bevorzugt sind.

Durch Variation des pH-Wertes kann ein Mittel zur Verfügung gestellt werden, das universell für jede Haarstruktur, gegebenenfalls unter zusätzlicher Wärmeeinwirkung, geeignet ist. Die Mittel sind entweder sauer (Sulfit, Bisulfit und Mercaptocarbonsäureester) oder alkalisch (Alkali- und Ammoniumsalze von Mercaptocarbonsäuren) eingestellt. Als Alkalisierungsmittel bzw. als Mittel zur Einstellung des pH-Wertes von z.B. 7 bis 10, insbesondere 7 bis 8,5 kommen insbesondere Ammoniak, Natronlauge, organische Amine, z.B. Monoethanolamin aber auch alle anderen wasserlöslichen, physiologisch verträglichen, basisch reagierenden, organischen oder anorganischen Salze, wie z.B. Ammonium- oder Alkalicarbonat und Ammonium- oder Alkalihydrogencarbonat in Betracht. Ist das Mittel sauer (z.B. pH = 6,5 bis 6,9) eingestellt, können Ester von Mercaptocarbonsäuren, z.B. Monothioglykolsäureglykolester oder -glycerinester, vorzugsweise Mercaptoacetamide oder 2-Mercaptopropionsäureamide, in einer Konzentration von 2 bis 14 Gew.%; oder die Salze der schwefeligen Säure, z.B. Natrium-, Ammonium- oder Monoethanolammoniumsulfit, in einer Konzentration von 3 bis 8 Gew.% (berechnet als SO₂), verwendet werden.

Die bei dem erfindungsgemäßen Verfahren verwendeten Dauerwellmittel können in Form einer wässrigen Lösung oder einer Emulsion sowie in verdickter Form auf wässriger Basis, insbesondere als Creme, Gel oder Paste, vorliegen. Besonders bevorzugt liegen sie in viskoser Form mit einer Konsistenz vor, welche es ermöglicht, das Haar im Sitzen und nicht wie gewöhnlich im Waschbecken zu behandeln, z.B. eine viskose Zubereitung mit einer Viskosität von 100 bis 10.000, vorzugsweise 300 bis 1000 mPa s bei 25 °C. Die Viskositätsangaben beziehen sich auf die Messung mit einem Haake RotationsViskosimeter Typ VT 501 bei einer Schergeschwindigkeit von 64,5 pro Sekunde. Ebenfalls ist es möglich, diese Mittel unter Druck in Aerosoldosen abzufüllen und daraus als Aerosolschaum zu entnehmen.

Den Dauerwellmitteln können zur Wirkungssteigerung sogenannte Quell- und Penetrationsstoffe, wie z.B. Harnstoff, mehrwertige Alkohole, Ether, Melamin, Alkali- oder Ammoniumthiocyanat, Isopropanol, Dipropylenglykolmonomethylether, 2-Pyrrolidon, Imidazolidin-2-on oder 1-Methyl-2-pyrrolidon in einer Menge von etwa 0,5 bis 50 Gew.%, vorzugsweise 2 bis 30 Gew.% sowie zur Vermeidung einer Überkrausung der Haare Dithioverbindungen, beispielsweise Dithiole, Dithiodiglykolsäure, Dithiomilchsäure oder die jeweiligen Salze der Dithiole zugesetzt werden.

Bei der Anwendung kann das Haar zunächst mit einem Shampoo gewaschen und danach mit Wasser gespült werden. Anschließend kann das handtuchtrockene Haar in einzelne Strähnen aufgeteilt werden, wobei nur einzelne Strähnen behandelt werden, wenn nur eine partielle Rasta-Frisur erstellt werden soll. Sodann wird das Haar mit einer ausreichenden Menge, vorzugsweise 60 bis 120 Gramm, des Mittels behandelt. Nach einer ausreichenden Einwirkungszeit, welche je nach Haarbeschaffenheit, dem pH-Wert und der Wirksamkeit des Dauerwellmittels sowie in Abhängigkeit von der Anwendungstemperatur 2 bis 30 Minuten (5 bis 30 Minuten ohne Wärmeeinwirkung; 2 bis 20 Minuten mit Wärmeeinwirkung) beträgt, wird das Haar mit Wasser gespült, wobei eher kürzere Einwirkzeiten bevorzugt sind, z.B. maximal 15 oder maximal 10 Minuten ohne Wärmeeinwirkung bzw. maximal 10 oder maximal 7 Minuten mit Wärmeeinwirkung.

### Zusammensetzung B

Die Aufbringung des die Haftung der Haare aneinander bewirkenden Stoffes kann mittels einer zweiten Zusammensetzung B erfolgen. Geeignete Stoffe sind z.B. Wachse, wachsartige Stoffe und/oder adhesive Polymere. Wachse und wachsartige Stoffe sind insbesondere solche Stoffe mit Eigenschaften gemäß der Definition für Wachse in Ullmanns Encyklopädie der technischen Chemie, 4. Auflage, Band 24, Seite 3. Demnach sind Wachse bei 20°C knetbar, fest bis brüchig hart, grob- bis feinkristallin, durchscheinend bis opak, jedoch nicht glasartig, über 40°C ohne Zersetzung schmelzend, schon wenig oberhalb ihres Schmelzpunktes verhältnismäßig niedrigviskos, stark temperaturabhängig in Konsistenz und Löslichkeit und unter leichtem Druck polierbar. Im Folgenden werden die Begriffe Wachse und wachsartige Stoffe synonym verwendet. Als adhesive Polymere sind solche geeignet, die dem Haar einen klebrigen Griff verleihen, feststellbar durch Halbseitenversuche von polymerbehandeltem Haar gegenüber unbehandeltem Haar.

Geeignete hydrophobe Wachse sind tierische, pflanzliche, mineralische und synthetische Wachse, mikrokristalline Wachse, makrokristalline Wachse, feste Paraffine, Petrolatum, Vaseline, Ozokerit, Montanwachs, Fischer-Tropsch-Wachse, Polyolefinwachse z.B. Polybuten, Bienenwachs, Wollwachs und dessen Derivate wie z.B. Wollwachsalkohole, Candelillawachs, Carnaubawachs, Japanwachs, gehärtete Fette, Fettsäureester und Fettsäureglyceride mit einem Erstarrungspunkt von jeweils oberhalb 40°C, Polyethylenwachse und Silikonwachse. Die Wachse haben einen Erstarrungspunkt oberhalb 40°C, vorzugsweise oberhalb 55 °C. Die Nadelpenetrationszahl (0,1 mm, 100 g, 5 s, 25°C; nach DIN 51 579) liegt vorzugsweise im Bereich von 2 bis 70, insbesondere von 3 bis 40. Besonders bevorzugt kleiner 20 aufweist. Besonders bevorzugt sind Carnaubawachs und Ceresin sowie deren Gemisch. Die Wachse bzw. wachsartigen Stoffe sind in der Zusammensetzung B vorzugsweise in einer Menge von 4 bis 50 Gew.%, besonders bevorzugt von 8 bis 30 Gew.%, insbesondere von 10 bis 25 Gew.% enthalten.

Die einen die Haftung der Haare aneinander bewirkenden Stoff enthaltende Zusammensetzung B kann in verschiedenen Applikationsformen vorliegen, insbesondere als Lotion, Gel, Aerosolspray, Non-Aerosol Pumpspray, Aerosolschaum, Non-Aerosol Schaumprodukt oder als festes oder halbfestes Wachsprodukt in Tiegel oder Stiftform.

In einer besonders bevorzugten Ausführungsform ist Zusammensetzung B eine wässrige Emulsion eines hydrophoben Wachses und enthält neben dem Wachs mindestens einen Emulgator und Wasser, wobei die Bezeichnung Emulsion jegliche Dispersion in Wasser umfaßt, also auch solche, bei denen es sich streng genommen um Suspensionen fester Wachspartikel in Wassser handelt. Die Emulgatoren sind vorzugsweise in einer Menge von 0,5 bis 50 Gew.%, besonders bevorzugt von 3 bis 40 Gew.%, insbesondere von 20 bis 35 Gew.% enthalten. Bevorzugte Emulgatoren sind solche aus der Klasse der nichtionischen Tenside. Geeignet sind z.B.
- Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin und
- Anlagerungsprodukte von 5 bis 60 mol Ethylenoxid an Rizinusöl oder an gehärtetes (hydriertes) Rizinusöl
- Mono-, Di- und/oder Triester der Phosphorsäure mit Anlagerungsprodukten von 2 bis 30 mol Ethylenoxid an C8-bis C22-Fettalkohole.

In einer besonders bevorzugten Ausführungsform ist die Summe der Emulgatoren in einer größeren Menge vorhanden als die Summe der Wachse d.h. das Gewichtsmengenverhältnis Emulgator:Wachs ist größer 1:1, vorzugsweise bis 5:1, besonders bevorzugt von 1,5:1 bis 3:1. Diese Ausführungsform zeichnet sich durch eine Kombination einzigartiger Wirkungen aus. Direkt nach dem Aufbringen auf das Haar ist die Klebrigkeit (Adhesionskraft) der Haare sehr hoch, was die Erstellung der Rasta-Frisur sehr erleichtert. Nach dem Trocknen reduziert sich die Klebrigkeit stark, was zu einem verbesserten, angenehmeren Griff führt. Eine geringe, nicht unangenehme Restadhesion verbleibt und bewirkt, dass fertige Rasta-Strähnen lose aneinander haften können, was neue Frisurgestaltungsmöglichkeiten eröffnet. Der besonders vorteilhafte Effekt der sich selbstätig abschwächenden Adhesionskraft ist besonders stark ausgeprägt in Kombination mit Silica als die Rauheit der Haaroberfläche erhöhendem Stoff. Ein weiterer, besonders vorteilhafter Effekt ist die gute, rückstandslose Entfernbarkeit des hydrophoben Wachses, insbesondere bei Anwendung der unten beschriebenen Zusammensetzung C zur Entfernung der Rasta-Frisur. Zusammensetzung B liegt idealerweise in fluider, d.h. flüssiger oder zähflüssiger Form vor und wird vorzugsweise über eine Pumpvorrichtung in fluider Form, über eine Sprühvorrichtung als Spray oder mit einer Vorrichtung zum Verschäumen als Schaum ausgebracht. Hinsichtlich der weiteren Bestandteile für Aerosol- und Non-Aerosol-Ausführungsformen wird auf die obigen Angaben bezüglich Zusammensetzung A verwiesen.

Als Wachse können aber auch hydrophile Wachse eingesetzt werden, insbesonders hochmolekulare Polyethylenglykole (PEG). Die Polyethylenglykole sind bei Raumtemperatur (20-25°C) vorzugsweise wachsartig fest oder zumindest weichwachsartig mit Erstarrungstemperaturen ab etwa 30°C, vorzugsweise ab 40°C. Das Molekulargewicht beträgt vorzugsweise von 850 bis etwa 5000 g/mol, besonders bevorzugt von 1200 bis 3500 g/mol. Polyethylenglykole besitzen die allgemeine Formel H(OCH₂CH₂)ₙOH. Geeignete hochmolekulare Polyethylenglykole sind z.B. solche mit n = 19 bis 113, vorzugsweise mit n = 30 bis 79. Geeignete Polyethylenglykole haben die INCI-Bezeichnungen PEG-20 (n = 20), PEG-32 (n = 32), PEG-60 (n = 60), PEG-75 (n = 75), PEG-90 (n = 90) und PEG-100 (n = 100). Handelsprodukte weisen in der Regel eine Molekulargewichtsverteilung auf. Geeignete Handelsprodukte sind z.B. Polyglykol 1000, Polyglykol 1350, Polyglykol 1500, Polyglykol 3000 oder Polyglykol 4000 der Firma Clariant, wobei die Zahlenwerte annähernd das mittlere Molekulargewicht angeben.

In einer weiteren, bevorzugten Ausführungsform handelt es sich bei der die Haftung der Haare aneinander erhöhenden Zusammensetzung B um ein Mikroemulsionsgel. Die Mikroemulsion wird gebildet aus einer hydrophoben, bei Raumtemperatur (20°C) flüssigen Ölkomponente, Emulgatoren und Wasser. Zusätzlich kann ein adhesives Polymer enthalten sein. Als Emulgatoren kommen die oben genannten Emulgatoren in den oben genannten Mengen in Frage. Als Ölkomponente kann prinzipiell jedes dem Fachmann allgemein bekannte Öl eingesetzt werden. In Frage kommen pflanzliche oder tierische Öle, Mineralöle, Silikonöle oder deren Mischungen. Geeignete Silikonöle sind Polydimethylsiloxane, phenylierte Silikone, Polyphenylmethylsiloxane, Phenyltrimethicone, Poly(C1-C20)alkylsiloxane, Alkylmethylsiloxane. Geeignet sind weiterhin Kohlenwasserstofföle, z.B. Paraffin- oder Isoparaffinöle, Squalan, Öle aus Fettsäuren und Monoalkoholen oder Polyolen, insbesondere Fettsäureester und Fettsäuretriglyceride. Geeignete pflanzliche Öle sind z.B. Sonnenblumenöl, Kokosöl, Rizinusöl, Lanolinöl, Jojobaöl, Maisöl, Sojaöl. Besonders bevorzugt sind Kohlenwasserstofföle, insbesonders Mineralöle (Paraffinum liquidum) und Fettsäuretriglyceride, wobei die Fettsäuren mindestens 8 C-Atome aufweisen. Die Ölkomponente ist vorzugsweise in einer Menge von 0,1 bis 25 Gew.%, besonders bevorzugt von 1 bis 10 Gew.% enthalten.
Die adhesiven Polymere sind vorzugsweise in einer Menge von 0,1 bis 25 Gew.%, besonders bevorzugt von 1 bis 10 Gew.% enthalten. Bei den adhesiven Polymeren handelt es sich vorzugsweise um Homo- oder Copolymere auf Acrylat- und/oder Acrylamidbasis, d.h. um Polymere, bei denen mindestens ein Monomer ausgewählt ist aus Acrylsäure, Methacrylsäure, Acrylsäureestern, Methacrylsäureestern, Acrylamiden, Methacrylamiden, Alkylacrylamiden und Alkylmethacrylamiden, wobei die Alkylgruppen vorzugsweise 1, 2, 3, 4 oder 5 C-Atome umfassen.

### Rasta-Reinigung

Zur Pflege der temporären Rasta-Frisur empfiehlt sich eine möglichst schonende Reinigung ohne große mechanische Beanspruchung der Rastasträhnen. Eine schonende Reinigung kann dadurch erfolgen, dass das Haar angefeuchtet wird und eine haarreinigende Zusammensetzung, welche mindestens ein waschaktives Tensid enthält, auf das Haar oder auf einem Schwamm aufgebracht und auf dem Haar vorsichtig, z.B. durch Tupfen mit einem Schwamm verteilt wird. Anschließend wird die Reinigungszusammensetzung wieder vom Haar entfernt, was durch abspülen mit Wasser oder durch abtupfen mit dem gegebenenfalls zwischendurch mehrfach ausgespültem Schwamm erfolgt. Der Schwamm hat vorzugsweise eine konkave Form, wobei die konkave Form in etwa der Kopfform entspricht. Die Aufbringung, Verteilung und/oder Entfernung der Reinigungszusammensetzung auf dem Haar erfolgt mit der konkaven Innenseite des Schwamms.

### Rasta-Entfernung

Die Entfernung der erfindungsgemäß erstellten temporären Rasta-Frisur kann dadurch erfolgen, dass das Haar angefeuchtet wird, eine Remover-Zusammensetzung C auf dem Haar aufgebracht und verteilt wird und die Rastasträhnen geöffnet und/oder entwirrt werden. Zum Schluß kann die Zusammensetzung C aus dem Haar ausgespült werden.

Die Zusammensetzung C enthält mindestens einen die Naßkämmbarkeit erhöhenden Stoff. Dieser Stoff ist vorzugsweise in einer Menge von 0,01 bis 10, besonders bevorzugt von 0,05 bis 5 Gew.% enthalten.Geeignet sind z.B. kationische Polymere, kationische Tenside, Aminoxide, kationische Silikonverbindungen, Ölkomponenten, Fettalkohole und Glycerinmonofettsäureester. Geeignete kationische Tenside sind Tenside, welche eine quaternäre Ammoniumgruppe und mindestens einen organischen Rest mit mindestens 6 C-Atomen enthalten. Geeignete kationische Tenside können durch die allgemeine Formel dargestellt werden,

N⁽⁺⁾R¹R²R³R⁴ X⁽⁻⁾

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens eine der Gruppen mindestens 6 C-Atome aufweist und X⁻ ein Anion darstellt, beispielsweise ein Halogen, Acetat, Phosphat, Nitrat oder Alkylsulfat, vorzugsweise ein Chlorid. Die aliphatischen Gruppen können zusätzlich zu den C-Atomen und den Wasserstoffatomen auch Querverbindungen oder andere Gruppen wie beispielsweise weitere Aminogruppen enthalten.

Die Gruppen können linear, verzweigt oder cyclisch sein. Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, z.B. Alkylmethylaminoxide oder Alkylaminoethyldimethylaminoxide min mindestens 6 C-Atomen in der Alkylgruppe. Besonders bevorzugt ist Cetyltrimethylammoniumchlorid.

Als kationische Polymere im Sinne der vorliegenden Erfindung werden solche Polymere verstanden, welche mindestens eine kationische oder durch Protonierung kationisierbare Gruppe enthalten. Kationische Gruppen sind beispielsweise quaternäre Amingruppen, kationisierbare Gruppen sind beispielsweise primäre, sekundäre oder tertiäre Amingruppen. Die kationischen Polymere können Homo- oder Copolymere sein, wobei die kationischen bzw. kationisierbaren Gruppen entweder in der Polymerkette oder vorzugsweise als Substituent an einem oder mehreren der Monomeren enthalten sind.

Geeignete Monomere des kationischen Polymers, welche kationisierbare Gruppen aufweisen, sind ungesättigte, radikalisch polymerisierbare Verbindungen, welche mindestens eine neutralisierte oder nicht neutralisierte basische Gruppe tragen. Als basische Gruppen kommen insbesondere primäre, sekundäre oder tertiäre Amine in Betracht, wobei das Aminstickstoffatom auch Teil eines Ringes sein kann. Beispiele für derartige Monomere sind Mono- und Dialkylaminoalkylacrylate oder -methacrylate. Die Alkylgruppen dieser Monomere sind vorzugsweise niedere Alkylgruppen wie z.B. C1- bis C7-Alkylgruppen, besonders bevorzugt C1- bis C4-Alkylgruppen.

Geeignete Monomere, welche quaternäre Amingruppen aufweisen, sind ungesättigte, radikalisch polymerisierbare Verbindungen, welche mindestens eine quaternäre Amingruppe tragen, insbesondere ammoniumsubstituierte Vinylmonomere oder quaternisierte Derivate von Carboxyvinylmonomeren wie z.B. quaternisierte Acrylamide oder Methacrylamide. Beispiele hierfür sind Acrylamidoalkyltrialkylammoniumhalogenide oder Methacrylamidoalkyltrialkylammoniumhalogenide, Trialkylmethacryloxyalkylammoniumhalogenide, Trialkylacryloxyalkylammoniumhalogenide, Dialkyldiallylammoniumhalogenide oder quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Bevorzugt sind Acrylamidopropyltrimethylammoniumchlorid und Methacrylamidopropyltrimethylammoniumchlorid.

Das kationische Polymer kann gegebenenfalls mit neutralen Comonomeren polymerisiert sein, die weder kationische noch kationisierbare Gruppen enthalten. Derartige neutrale Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geignete kationische Polymere sind z.B. Polyvinylpyrrolidon/Dimethylaminoethylmethacrylat Copolymere, Copolymere aus Polyvinylpyrrolidon und Imidazoliminmethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, quaternierte Ammoniumsalze der Hydroxyethylcellulose (INCI-Bezeichnung: Polyquaternium-10 oder Polyquaternium-24), kationische Guarderivate, Vinylpyrrolidon/Methacrylamidopropyltrimethylammoniumchlorid Copolymere oder diquaternäre Polydimethylsiloxane (INCI-Bezeichnung: Quaternium-80), Stearyldimethylammonium-hydroxyethylcellulose, Methacryloyl Ethyl Betain/Methacrylat Copolymere, Polymethacrylamidopropyl Trimonium Chlorid, Polyquaternium-2, Polyquaternium-6, Polyquaternium-7, Polyquaternium-18, Polyquaternium-22, Polyquaternium-27, Polyquaternium-39 sowie Polymere mit Siloxaneinheiten, z.B. Polyquaternium-41 oder Polyquaternium-42.

Ein geeignetes kationisches Polymer ist Chitosan oder ein Chitosanderivat, welches mit einer kosmetisch verträglichen Säure neutralisiert worden ist. Bei der Säure kann es sich um eine organische oder anorganische Säure handeln, Z.B. Ameisensäure, Weinsäure, Äpfelsäure, Maleinsäure, Fumarsäure, Pyrrolidoncarbonsäure, Zitronensäure, Milchsäure, Schwefelsäure, Essigsäure, Salzsäure, Phosphorsäure u.a..

Als Chitosanderivate kommen beispielsweise quaternierte, alkylierte oder hydroxyalkylierte Derivate, beispielsweise Hydroxyethyl-, Hydroxypropyl- oder Hydroxybutylchitosan in Betracht. Das Chitosan oder Chitosanderivat hat vorzugsweise ein Molekulargewicht von 20.000 bis ca. 5 Millionen g/mol. Geeignet ist beispielsweise ein niedermolekulares Chitosan mit einem Molekulargewicht von 30.000 bis 70.000 g/mol oder ein hochmolekulares Chitosan mit einem Molekulargewicht von 300.000 bis 700.000 g/mol. Der bevorzugte Entacetylierungsgrad des Chitosans liegt zwischen 10 und 99%. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel

G-O-B-N⁺R^{a}R^{b}R^{c} X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X⁻ ist ein übliches Gegenanion, z.B. ein Halogen, Acetat, Phosphat, Nitrat oder Alkylsulfat, vorzugsweise ein Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar R vertrieben und hat die INCI-Bezeichnung Guar Hydroxypropyltrimonium Chloride.

Bevorzugt sind solche Polymere, die eine ausreichende Löslichkeit in Wasser, C1-4-Alkoholen oder deren Gemischen besitzen, um in dem erfindungsgemäßen Mittel in vollständig gelöster Form vorzuliegen. Die kationische Ladungsdichte beträgt vorzugsweise 1 bis 7 meq/g.

Geeignete kationaktive Silikonvberbindungen weisen vorzugsweise entweder mindestens eine Aminogruppe oder mindestens eine Ammoniumgruppe auf. Geeignete Silikonpolymere mit Aminogruppen sind unter der INCI-Bezeichnung Amodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel

R⁸R⁹R¹⁰Si- (OSiR¹¹R¹²)x- (OSiR¹³Q)y-OSiR¹⁴R¹⁵R¹⁶

R⁸, R⁹, R¹⁴ und R¹⁵ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹⁰ und R¹⁶ sind unabhängig voneinander gleich oder verschieden und bedeuten -(CH₂)ₐ-NH₂ mit a gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹¹, R¹² und R¹³ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1- bis C4-Alkyl, insbesondere Methyl;
Q bedeutet -A-NR¹⁷R¹⁸, oder -A-N⁺R¹⁷R¹⁸R¹⁹ wobei A für eine divalente C1- bis C20-Alkylenverbindungsgruppe steht, welche auch O- und N-Atome sowie OH-Gruppen enthalten kann, und
R¹⁷, R¹⁸ und R¹⁹ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind -(CH₂)₃-NH₂, -(CH₂)₃NHCH₂CH₂NH₂, -(CH₂)₃OCH₂CHOHCH₂NH₂ und -(CH₂)₃N(CH₂CH₂OH)₂, -(CH₂)₃-NH₃⁺ und -(CH₂)₃OCH₂CHOHCH₂N⁺(CH₃)₂R²⁰, wobei R²⁰ ein C1- bis C22-Alkylrest ist, der auch OH-Gruppen aufweisen kann.
X bedeutet eine Zahl zwischen 1 und 10.000, vorzugsweise zwischen 1 und 1.000;
Y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Geeignete Silikonpolymere mit zwei enständigen quaternären Ammoniumgruppen sind unter der INCI-Bezeichnung Quaternium-80 bekannt. Hierbei handelt es sich um Dimethylsiloxane mit zwei endständigen Aminoalkylgruppen. Geeignete quaternäre Aminosilikone sind solche der allgemeinen Formel

R²¹R²²R²³N⁺-A-SiR⁸R⁹ - (OSiR¹¹R¹²)ₙ -OSiR⁸R⁹-A-N⁺R²¹R²²R²³ 2X⁻

wobei A für eine divalente C1- bis C20-Alkylenverbindungsgruppe steht, welche auch O- und N-Atome sowie OH-Gruppen enthalten kann, eine bevorzugte Gruppe ist -(CH₂)₃OCH₂CHOHCH₂N⁺(CH₃)₂R²⁰, wobei R²⁰ ein C1- bis C22-Alkylrest ist, der auch OH-Gruppen aufweisen kann;
R⁸, R⁹, R¹¹ und R¹² haben die gleiche Bedeutung wie oben angegeben und sind vorzugsweise Methyl;
R²¹, R²², und R²³ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise von 10 bis 100. Derartige diquaternäre Polydimethylsiloxane werden von der Firma GOLDSCHMIDT/Deutschland unter den Handelsnamen Abil® Quat 3270, 3272 und 3274 vertrieben.

Weitere geeignete kationische Polymere sind kationisch modifizierte Proteinderivate oder kationisch modifizierte Proteinhydrolysate und sind beispielsweise bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein oder Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein.

Geeignete kationisch derivatisierte Proteinhydrolysate sind Substanzmischungen, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Proteine, die als Ausgangsstoffe für die Proteinhydrolysate dienen, können sowohl pflanzlicher als auch tierischer Herkunft sein. Übliche Ausgangsstoffe sind beispielsweise Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000. Vorteilhafterweise enthalten die kationisch derivatisierten Proteinhydrolysate eine oder zwei lange C8- bis C22-Alkylketten und entsprechend zwei oder eine kurze C1- bis C4-Alkylketten. Verbindungen, die eine lange Alkylkette enthalten, sind bevorzugt.

Geeignete Fettalkohole sind Alkylalkohole mit 8 bis 22 C-Atomen, z.B. Myristyl-, Cetyl- oder Stearylalkohol oder deren Mischungen. Die Alkylgruppe kann linear oder verzweigt sein.

Geeignete Glycerinmonofettsäureester sind die Monoester von Glycerin und den genannten Fettalkoholen.

Geeignete Ölkomponenten sind beispielsweise pflanzliche oder tierische Öle, Mineralöle, Silikonöle oder deren Mischungen. Geeignete Silikonöle sind Polydimethylsiloxane, phenylierte Silikone, Polyphenylmethylsiloxane, Phenyltrimethicone, Poly(C1-C20)alkylsiloxane, Alkylmethylsiloxane. Geeignet sind weiterhin Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl. Besonders bevorzugt sind Kohlenwasserstofföle, insbesonders Mineralöle (Paraffinum liquidum).

### Zusammensetzung C

Eine bevorzugte Ausführungsform für die Remover-Zusammensetzung ist eine O/W-Emulsion und enthält
(A) ca. 0,1 bis 10 Gew.%, vorzugsweise 0,3 bis 5 Gew.% mindestens eines kationaktiven Haarpflegewirkstoffs, vorzugsweise ausgewählt aus kationischen Tensiden, kationischen Polymeren und kationischen Silikonverbindungen,
(B) ca. 0,5 bis 15 Gew.%, vorzugsweise 1 bis 10 Gew.% mindestens einer hydrophoben Fett- oder Ölkomponente, vorzugsweise ausgewählt aus Fettalkoholen, Fettalkoholestern, natürlichen Ölen, Mineralölen und Silikonölen,
(C) ca. 75 bis 98 Gew.%, vorzugsweise 80 bis 95 Gew.% Wasser sowie gegebenenfalls Hilfs- und Zusatzstoffe wie z.B. Verdickungsmittel, mehrwertige Alkohole, Parfüm, Farbstoffe, Konservierungsmittel, Säuren, haarpflegende Wirkstoffe.

Zusammensetzung C kann auch in Form eines Aerosolproduktes, insbesondere eines Aerosolschaums vorliegen. Für die hierfür erforderlichen zusätzlichen Bestandteile wie Lösungs- und Treibmittel, wird auf die obigen Angaben bezüglich Zusammensetzung A verwiesen.

### Mehrkomponenten-Kits

Gegenstand der Erfindung sind auch Mehrkomponenten-Kits zur Ausführung des erfindungsgemäßen Verfahrens.
Ein erfindungsgemäßes Kit enthält mindestens zwei Komponenten, ausgewählt aus
- einer oben näher beschriebenen Zusammensetzung, welche die Rauheit der Haare erhöht,
- einer oben näher beschriebenen Zusammensetzung, welche eine Haftung der Haare aneinander bewirkt,
- einem mechanischen Toupierhilfsmittel, z.B. einem Kamm, insbesondere einem Toupierkamm, einer Bürste, einem Striegel oder einer automatischen Toupier- oder Flechtvorrichtung für Haare,
- einem formstabilen, konkav geformten Schwamm,
- eine mindestens ein waschaktives Tensid enthaltende Reinigungszusammensetzung,
- eine mindestens einen die Naßkämmbarkeit erhöhenden Stoff enthaltende Zusammensetzung, vorzugsweise ausgewählt aus kationischen Polymeren, kationischen Tensiden, Aminoxiden, kationischen Silikonverbindungen, Ölkomponenten, Fettalkoholen und Fettalkoholestern und/oder
- einen Datenträger, auf welchem die Verfahrensschritte des erfindungsgemäßen Verfahrens ganz oder teilweise in graphischer, bildlicher und/oder sprachlicher Form aufgezeichnet sind.

Ein erfindungsgemäßes Kit enthält vorzugsweise mindestens eine erste Komponente, bei der es sich um eine Zusammensetzung handelt, welche die Rauheit der Haare erhöht und mindestens eine zweite Komponente, bei der es sich um eine Zusammensetzung handelt, welche eine Haftung der Haare aneinander bewirkt und entweder eine Emulsion aus mindestens einem hydrophoben Wachs, mindestens einem Emulgator und Wasser oder mindestens ein adhesives Polymer oder mindestens ein hydrophiles Wachs enthält. Die Zusammensetzungen sind oben näher beschrieben. Die erste Komponente umfaßt vorzugsweise eine Zusammensetzung welche mindestens einen Stoff enthält, der ausgewählt ist aus Silica, Silikaten, Aluminaten, Tonerden, Mica, Salzen, Metalloxiden, Mineralen und Polymeren. Besonders bevorzugt ist entweder eine wässrige Salzlösung, die mit einer Sprühvorrichtung auf das Haar aufgesprüht werden kann oder eine schaumbildende Zubereitung, wobei diese Zubereitung Silica, mindestens einen schaumbildenden Emulgator, Wasser und entweder mindestens ein Aerosol-Treibmittel enthält oder in Kombination mit einer mechanischen Vorrichtung zum Verschäumen vorliegt. Die zweite Komponente umfaßt vorzugsweise eine Emulsion aus mindestens einem hydrophoben Wachs, mindestens einem Emulgator und Wasser.

Die Zusammensetzung A, B und/oder C können zusätzlich 0,01 bis 15 Gew.%, vorzugsweise 0,5 bis 10 Gew.% mindestens eines synthetischen oder natürlichen nichtionischen filmbildenden Polymers enthalten. Bevorzugt sind insbesondere solche Polymere, die eine ausreichende Löslichkeit in Wasser oder Wasser/Alkohol-Gemischen besitzen, um in den Zusammensetzungen in vollständig gelöster Form vorzuliegen. Unter filmbildenden Polymeren werden erfindungsgemäß solche Polymere verstanden, die bei Anwendung in 0,01 bis 5%-iger wässriger, alkoholischer oder wässrig-alkoholischer Lösung in der Lage sind, auf dem Haar einen Polymerfilm abzuscheiden. Geeignete synthetische, nichtionische filmbildende, haarfestigende Polymere sind Homo- oder Copolymere, die aus mindestens einem der folgenden Monomere aufgebaut sind:
Vinylpyrrolidon, Vinylcaprolactam, Vinylester wie z.B. Vinylacetat, Vinylalkohol, Acrylamid, Methacrylamid, Alkylund Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.
Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und
Vinylacetat, Terpolymere aus Vinylpyrrolidon, Vinylacetat und
Vinylpropionat, Polyacrylamide; Polyvinylalkohole sowie Polyethylenglykol/Polypropylenglykol-Copolymere. Besonders bevorzugt sind Polyvinylpyrrolidon und Copolymere von Vinylpyrrolidon und nichtionischen Comoneren, beispielsweise
Polyvinylpyrrolidon/Vinylacetat Copolymere. Geeignete natürliche filmbildende Polymere sind z.B. Cellulosederivate, insbesondere Hydroxyalkylcellulosen, z.B. Hydroxypropylcellulose.

Die Zusammensetzungen können darüber hinaus die für Haarbehandlungsmittel üblichen Zusatzbestandteile enthalten, z.B. Parfümöle in einer Menge von 0,01 bis 0,5 Gew.%; Trübungsmittel, z.B. Ethylenglykoldistearat, in einer Menge von etwa 0,2 bis 5,0 Gew.%; Konservierungsmittel, z.B. Parabene in einer Menge von 0,01 bis 1,0 Gew.%; Puffersubstanzen, z.B. Natriumcitrat oder Natriumphosphat, in einer Menge von 0,1 bis 1,0 Gew.%; Pflegestoffe, z.B. Pflanzen- und Kräuterextrakte, Protein- und Seidenhydrolysate, Lanolinderivate, in einer Menge von 0,1 bis 5 Gew.%; physiologisch verträgliche Silikonderivate, z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; direktziehende Haarfarbstoffe, Glanzgeber, Vitamine, Weichmacher und rückfettende Agenzien.

Die nachfolgenden Beispiele sollen den Gegenstand der Erfindung näher erläutern.

### Beispiele

### Rasta-Erstellung

Die Haare werden mit einem üblichen Shampoo, vorzugsweise einem nicht konditionierendem Neutralshampoo gewaschen. Es werden ca. 6 bis 15 g (je nach Haarlänge) der folgenden Zusammensetzung A gleichmäßig im Haar verteilt:

### Zusammensetzung A1 (Aerosolschaum):

| | |
|---|---|
| 1 g | Fluisil® 300 (Silica) |
| 0,8 g | Rewoteric® AM CAS (Cocamidopropyl Hydroxysultaine, 50%ig in Wasser) |
| 0,4 g ad 100 g | Oramix® NS 10 (Decyl Glucoside, 55%ig in Wasser) Wasser |

Abgefüllt mit Propan/Butan im Verhältnis 96:4 in einer Aerosoldose mit Schaumkopf.

Alternativ kann ein Haarspray mit der Zusammensetzung A2 in das trockene und zu verklettende Haar gesprüht werden.

### Zusammensetzung A2:

| | |
|---|---|
| 2 g | Fluisil® 300 (Silica) |
| ad 100 g | Ethanol |

Abgefüllt mit Propan/Butan (4,8 bar) im Verhältnis 96:4 in einer Aerosoldose mit Sprühkopf.

Nach dem Trocknen der Haare wird ein Mittelscheitel gezogen, vorzugsweise in Form eines Zickzack-Scheitels und die Haare werden vom Nacken her in gleichmäßig breite Abteilungen unterteilt. Die Abteilungen werden in gleichmäßig große Quadrate oder Dreiecke unterteilt. Zur Erstellung einer nur partiellen Rasta-Frisur werden nur einzelne Partien der Haare mit einer Zusammensetzung A1 oder A2 behandelt. Die Haarsträhnen werden vom Ansatz her unter leichten Drehbewegungen zu den Spitzen leicht toupiert (back combing). Die Strähnen werden beim Bearbeiten in der gewünschten Fallrichtung der Dreads gehalten. Nach dem Toupieren aller Strähnen des ganzen Kopfes werden die Strähnen einzeln mit einer der folgenden Sealer-Zusammensetzungen B1 bis B5 behandelt und in Form gedreht.

### Zusammensetzung B1 (Pumpspray) :

| | |
|---|---|
| 17,8 g | PEG-60 Hydrogenated Castor Oil |
| 11,9 g | Carnaubawachs |
| 8,9 g | PEG-40 Hydrogenated Castor Oil |
| ad 100 g | Wasser |

Abgefüllt in einer Verpackung mit Pumpsprühvorrichtung.

### Zusammensetzung B2:

| | |
|---|---|
| 7,8 g | PEG-60 Hydrogenated Castor Oil |
| 11,9 g | Carnaubawachs |
| 9,9 g | PEG-40 Hydrogenated Castor Oil |
| 10 g | Oleth-20 |
| ad 100 g | Wasser |

### Zusammensetzung B3 (Aerosolschaum)

| | |
|---|---|
| 20 g | PEG-60 Hydrogenated Castor Oil |
| 10 g | Carnaubawachs |
| 2,5 g | Glycerin (86%ig) |
| 2,5 g | Caprylic/Capric Triglyceride |
| 35 g | Wasser |
| ad 100 g | Ethanol |

Abgefüllt als Schaumaerosol mit Propan/Butan (4,8 bar) im Verhältnis 96:84 in einer Aerosoldose mit Schaumkopf.

### Zusammensetzung B4 (Mikroemulsions-Gel)

Zunächst wird eine Mikroemulsion hergestellt aus

| | |
|---|---|
| 10 g | Oleth-10 |
| 9 g | PEG-40 Hydrogenated Castor Oil |
| 10 g | Sucrose Cocoate (92,5%ig) |
| 10 g | Oleth-5 |
| 12 g | Caprylic/Capric Triglyceride |
| ad 100 g | Wasser |

### Das Gel wird hergestellt aus

| | |
|---|---|
| 40 g | o.g. Mikroemulsion |
| 10 g | Acrylates/t-Butylacrylamid Copolymer (Ultrahold® 8) |
| 1 g | 2-Aminobutanol |
| ad 100 g | Wasser |

### Zusammensetzung B5 (Gel-Wachs) :

| | |
|---|---|
| 94,5 g | Zusammensetzung B1 |
| 5 g | Acrylates/t-Butylacrylamid Copolymer (Ultrahold® 8) |
| 0,5 g | 2-Aminobutanol |
| 11,9 g | Carnaubawachs |
| ad 100 g | Wasser |

### Waschen der Rastas

Die Frisur wird mit der Brause oder unter der Dusche mit leichtem Wasserstrahl gut durchnässt. Ein mildes Shampoo wird auf die konkave Innenseite eines konkaven, feuchten Waschschwamms aufgetragen. Mittels Betupfen der Frisur (keine kreisenden Bewegungen) wird das Shampoo zum Schäumen gebracht. Es wird gründlich aber sanft abgespült. Nach dem Waschen können die Dreads unter Verwendung von Zusammensetzung B wieder nachgedreht werden.

### Lösen der Rastas

Nach dem Waschen wird eine kationische Kur der folgenden Zusammensetzung C in die feuchte Frisur eingearbeitet.

### Zusammensetzung C

| | |
|---|---|
| 6,0 g | Cetearylalkohol |
| 1,35 g | Vaseline |
| 1,2 g | Paraffinum Perliquidum |
| 1,0 g | Cetyltrimethylammoniumchlorid |
| 0,3 g | Lanolinalkohol |
| 0,15 g | Lanolin |
| 0,5 g | Silikonöl |
| 0,5 g | Citronensäure |
| ad 100 g | Wasser |

Nach einer Einwirkzeit von ca. 5 Minuten werden die Dreads gelöst, wobei die Kur im Haar verbleibt. Die Dreads werden einzeln, bei den Haarspitzen beginnend hin zum Haaransatz mit einem Griffkamm behutsam ausgekämmt. Nach dem Lösen der Dreads wird die Kur ausgespült. Anschließend kann eine gewünschte neue Frisur erstellt werden.

## Patentansprüche

1. Verfahren zur Erstellung einer temporären Rasta-Frisur, wobei vor, während oder nach der Frisurenerstellung mindestens ein Verfahrensschritt erfolgt, der ausgewählt ist aus
(1) Aufbringen mindestens eines die Rauheit der Haaroberfläche erhöhenden Stoffes oder mindestens einer das Toupieren der Haare erleichternden Zusammensetzung auf das Haar und
(2) Aufbringen mindestens eines die Haftung der Haare aneinander erhöhenden Stoffes auf das Haar und/oder auf die Haarspitzen.

2. Verfahren nach Anspruch 1, **dadurch gekennzeichnet, dass** nach Aufbringen des die Rauheit der Haaroberfläche erhöhenden Stoffes oder der das Toupieren der Haare erleichternden Zusammensetzung die Haare strähnchenweise verklettet werden, entweder durch leichtes Toupieren (back combing), oder durch ein- oder mehrfaches Verdrehen, Teilen und Auseinanderziehen der geteilten Strähnchen, oder durch eine Kombination von Verdrehen der Strähnchen über die Längsachse und leichtem Toupieren der verdrehten Strähnchen.

3. Verfahren nach Anspruch 2, **dadurch gekennzeichnet, dass** zusätzlich der die Haftung der Haare aneinander erhöhende Stoff auf das Haar und/oder auf die Haarspitzen aufgebracht wird.

4. Verfahren nach Anspruch 3, **dadurch gekennzeichnet, dass** der die Haftung der Haare aneinander erhöhende Stoff ausgewählt ist aus Wachsen und adhesiven Polymeren.

5. Verfahren nach Anspruch 4, **dadurch gekennzeichnet, dass** der die Haftung der Haare erhöhende Stoff mittels einer Zusammensetzung B aufgebracht wird, welche eine Emulsion aus mindestens einem hydrophoben Wachs, mindestens einem Emulgator und Wasser enthält.

6. Verfahren nach einem der vorhergehenden Ansprüche, **dadurch gekennzeichnet, dass** der die Rauheit der Haaroberfläche erhöhende Stoff auf dem Haar in Form von Feststoffpartikeln haftet und mittels einer Zusammensetzung A aufgebracht wird, in welcher der Stoff bereits in Form von ungelösten Feststoffpartikeln vorliegt oder in welcher der Stoff in gelöster Form vorliegt und sich nach dem Aufbringen auf das Haar in Form von Feststoffpartikeln auf dem Haar abscheidet.

7. Verfahren nach Anspruch 6, **dadurch gekennzeichnet, dass** die Zusammensetzung A mindestens einen Stoff enthält, der ausgewählt ist aus, Silica, Silikaten, Aluminaten, Tonerden, Mica, Salzen, Metalloxiden, Mineralen und Polymeren.

8. Verfahren nach Anspruch 7, **dadurch gekennzeichnet, dass** die Zusammensetzung A schäumbar ist und Silica, mindestens einen schaumbildenden Emulgator, Wasser und entweder mindestens ein Aerosol-Treibmittel enthält oder in Kombination mit einer mechanischen Vorrichtung zum Verschäumen vorliegt.

9. Verfahren zur Pflege von temporären Rasta-Frisuren, wobei eine nach einem Verfahren gemäß einem der Ansprüche 1 bis 8 erstellte temporäre Rasta-Frisur gereinigt wird, indem eine mindestens ein waschaktives Tensid enthaltende Reinigungszusammensetzung auf das Haar aufgebracht, auf dem Haar verteilt und anschließend wieder vom Haar entfernt wird, wobei Aufbringung, Verteilung und/oder Entfernung der Zusammensetzung auf dem Haar mittels eines formstabilen konkaven Schwamms erfolgt.

10. Verfahren zur Entfernung von temporären Rasta-Frisuren, wobei eine nach einem Verfahren gemäß einem der Ansprüche 1 bis 8 erstellte temporäre Rasta-Frisur angefeuchtet wird, eine Zusammensetzung C auf dem Haar aufgebracht und verteilt wird, die Rastasträhnen geöffnet und/ oder entwirrt werden und wobei die Zusammensetzung C mindestens einen die Naßkämmbarkeit erhöhenden Stoff enthält.

11. Verfahren nach Anspruch 10, **dadurch gekennzeichnet dass** die Zusammensetzung C mindestens einen Stoff enthält, der ausgewählt ist aus kationischen Polymeren, kationischen Tensiden, Aminoxiden, kationischen Silikonverbindungen, Fett- oder Ölkomponenten und Glycerinmonofettsäureestern.

12. Verfahren nach Anspruch 11, **dadurch gekennzeichnet, dass** die Zusammsetzung C eine emulsionsförmige Haarkur ist, enthaltend
c1) mindestens einen kationaktiven Haarpflegewirkstoff, ausgewählt aus kationischen Tensiden, kationischen Polymeren und kationischen Silikonverbindungen,
c2) mindestens eine Fett- oder Ölkomponente und
c3) Wasser.

13. Verwendung von die Rauheit der Haaroberfläche erhöhenden Stoffen und/oder von das Toupieren der Haare erleichternden Zusammensetzungen und/oder von die Haftung der Haare aneinander erhöhenden Stoffen zur Erstellung temporärer Rasta-Frisuren.

14. Verwendung nach Anspruch 13, **dadurch gekennzeichnet, dass** die die Rauheit der Haaroberfläche erhöhenden Stoffe partikelförmige Feststoffe sind und/oder dass die die Haftung der Haare aneinander erhöhenden Stoffe ausgewählt sind aus Wachsen und adhesiven Polymeren.

15. Verwendung nach Anspruch 14, **dadurch gekennzeichnet, dass** die die Haftung der Haare aneinander erhöhenden Stoffe in Form einer Zusammensetzung eingesetzt werden, welche eine Emulsion aus mindestens einem hydrophoben Wachs, mindestens einen Emulgator und Wasser enthält.

16. Verwendung eines formstabilen, konkav geformten Schwamms zur Reinigung von temporären Rasta-Frisuren.

17. Verwendung einer Zusammensetzung, welche mindestens einen die Naßkämmbarkeit von Haaren verbessernden Stoff, ausgewählt aus kationischen Polymeren, kationischen Tensiden, Aminoxiden, kationischen Silikonverbindungen, Ölkomponenten, Fettalkoholen und Fettalkoholestern enthält, zur Entfernung von temporären Rasta-Frisuren.

18. Verwendung nach Anspruch 17, **dadurch gekennzeichnet, dass** die Zusammsetzung eine emulsionsförmige Haarkur ist, enthaltend
c1) mindestens einen kationaktiven Haarpflegewirkstoff, ausgewählt aus kationischen Tensiden, kationischen Polymeren und kationischen Silikonverbindungen,
c2) mindestens eine Fett- oder Ölkomponente und
c3) Wasser.

19. Mehrkomponenten-Kit zur Erstellung, Pflege und/oder Entfernung einer temporären Rasta-Frisur enthaltend mindestens zwei Komponenten, die ausgewählt sind aus
- einer Zusammensetzung, welche die Rauheit der Haare erhöht,
- einer Zusammensetzung, welche eine Haftung der Haare aneinander bewirkt,
- einem mechanischen Toupierhilfsmittel,
- einem formstabilen, konkav geformten Schwamm,
- eine mindestens ein waschaktives Tensid enthaltende Reinigungszusammensetzung,
- eine mindestens einen die Naßkämmbarkeit erhöhenden Stoff enthaltende Zusammensetzung und/oder
- einen Datenträger, auf welchem die Verfahrensschritte des erfindungsgemäßen Verfahrens ganz oder teilweise in graphischer, bildlicher und/oder sprachlicher Form aufgezeichnet sind.

20. Mehrkomponenten-Kit nach Anspruch 19, enthaltend mindestens eine erste Komponente, bei der es sich um eine Zusammensetzung handelt, welche die Rauheit der Haare erhöht und/oder das Toupieren der Haare erleichtert und mindestens eine zweite Komponente, bei der es sich um eine Zusammensetzung handelt, welche eine Haftung der Haare aneinander bewirkt und entweder eine Emulsion aus mindestens einem hydrophoben Wachs, mindestens einem Emulgator und Wasser oder mindestens ein adhesives Polymer oder mindestens ein hydrophiles Wachs enthält.

21. Kit nach Anspruch 19 oder 20, **dadurch gekennzeichnet, dass** die erste Komponente mindestens einen Stoff enthält, der ausgewählt ist aus Silica, Silikaten, Aluminaten, Tonerden, Mica, Salzen, Metalloxiden, Mineralen und Polymeren und/oder die zweite Komponente eine Emulsion aus mindestens einem hydrophoben Wachs, mindestens einen Emulgator und Wasser enthält.

22. Kit nach einem der Ansprüche 19 bis 21, **dadurch gekennzeichnet, dass** die erste Komponente entweder eine wässrige Salzlösung oder eine schaumbildende Zubereitung umfaßt, wobei die Zubereitung Silica, mindestens einen schaumbildenden Emulgator, Wasser und entweder mindestens ein Aerosol-Treibmittel enthält oder in Kombination mit einer mechanischen Vorrichtung zum Verschäumen vorliegt.

23. Kit nach einem der Ansprüche 19 bis 22, **dadurch gekennzeichnet, dass** der die Naßkämmbarkeit erhöhende Stoff ausgewählt ist aus kationischen Polymeren, kationischen Tensiden, Aminoxiden, kationischen Silikonverbindungen, Ölkomponenten, Fettalkoholen und Fettalkoholestern.

24. Zusammensetzung in Form einer fluiden, wässrigen Wachsemulsion zur Erstellung von temporären Rasta-Frisuren, enthaltend mindestens ein hydrophobes Wachs, mindestens einen Emulgator und Wasser, wobei die Menge an Emulgator größer ist als die Menge der Summe von Wachs und wachsartigen Stoffen.
